# EUROPEAN PATENT APPLICATION

(11) **EP 2 042 873 A1**
(43) Date of publication of application: **01.04.2009**
(21) Application number: 07117487.4
(22) Date of filing: 28.09.2007
(51) Int. Cl.: G01N 33/68

(54) **Means and methods for the detection of contrast medium-induced nephrotoxicity**

(71) Applicant: Roche Diagnostics GmbH, 68305 Mannheim (DE); F. Hoffmann-La Roche AG, 4070 Basel (CH)
(72) Inventor: The designation of the inventor has not yet been filed
(74) Representative: Dick, Alexander

(57) **Abstract**

The present invention relates to a method for diagnosing contrast medium-induced nephrotoxicty in a subject based on comparing the amount of urotensin II and/or adiponectin in a sample of said subject obtained after administration of the contrast medium to the amount of urotensin II and/or adiponectin in a sample of said subject prior to administration of the contrast medium. Further encompassed by the present invention arc a kit and a device for carrying out the method of the present invention.

## Description

The present invention relates to a method for diagnosing contrast medium-induced nephrotoxicty in a subject based on comparing the amount of urotensin II and/or adiponectin in a sample of said subject obtained after administration of the contrast medium to the amount of urotensin II and/or adiponectin in a sample of said subject prior to administration of the contrast medium. Further encompassed by the present invention are a kit and a device for carrying out the method of the present invention.

Worldwide, over 80 millions doses of iodinated contrast media are administered each year, e.g. for angiography or computed tomography, corresponding to approximately 8 million liters. This is one of the highest volumes of medical drugs used (Katzberg (2006), Kidney Int Suppl 100: S3-7). Contrast medium-induced nephrotoxicity (CIN) remains one of the most clinically important complications associated with the administration of contrast media. It is a common cause of hospital acquired acute renal failure and may require dialysis. Therefore, the condition of contrast medium-induced nephrotoxicity is both dangerous and cost intensive. Patients experiencing contrast medium-induced nephrotoxicity generally have more complications, more serious long term outcomes and a prolonged hospital stay than patients without CIN resulting in increased medical cost (Aspelin, P. (2004), Nephrotoxicity and the Role of Contrast Media, Radiation Medicine, 22(6): 377-378).

The exact mechanism behind CIN is not completely understood. It was suggested that causes of CIN are renal ischemia (by reducing blood flow or increasing oxygen demand) and, presumably, direct toxicity of the contrast medium to tubular epithelial cells. Moreover, the osmolality and the viscosity of the contrast medium are thought to play a role in CIN.

Moderate to severe chronic kidney disease (CKD) defined as estimated glomerular filtration rate < 60 ml/min per 1.73 m² (National Kidney foundation, CKD stages 3 and 4) is the most important risk factor of CIN. Therefore, these patients should be excluded from an examination that requires the administration of large doses of a contrast medium. Other major risk factor of CIN include older age, diabetes mellitus, intraarterial contrast medium administration, use of larger contrast medium doses, the concurrent use of nephrotoxic drugs, patient dehydration and any other condition associated with decreased effective circulation volume (Solomon, R. J. et al. (2007) Cardiac Angiography in Renally Impaired Patients. Circulation 112: 3189-3196.). However, also patients who do not have an apparent renal disease or dysorder are also at risk of suffering from CIN after the administration of a contrast medium.

To date, there is no optimal strategy to prevent CIN. Recent guidelines recommend intravenous volume expansion with a saline solution, use of low- or iso-osmolality contrast medium and limiting the volume of administered contrast medium (Solomon R., (2006) How to prevent contrast-induced nephrotoxicity and manage risk patients: Practical recommendations. Kidney Int 69: S51-S53). However, even when following these guidelines, there is still a significant risk of CIN after the administration of contrast media. Therefore, it is important to diagnose or predict the risk of CIN as early as possible after contrast medium administration in order to start an appropriate treatment.

However, the diagnosis of CIN is difficult. The diagnosis comprises clinical characteristics (such as anuria or oliguria) or the determination of an increase of substances that are obligatory excreted by urine (creatinine, urea, Cystatine C). Nowadays, CIN is defined by an increase of Serum Creatinine ≥ 0.5 mg/dL from the baseline within 45 -120 hours after administration of the contrast medium, a ≥ 25 % increase of Serum Creatinine or a ≥ 25 % decrease of the estimated glomerular filtration rate (GFR). Since it is difficult, time- and cost-intensive to determine the GFR, CIN is typically assessed via the changes of the creatinine concentration. However, the use of creatinine as a marker for CIN has been recently put into question as there is evidence that high-dose contrast media could interfere with tubular creatinine secretion. This may lead to an increase of creatinine and, therefore, may feign a decrease of GFR (Braeutigam, M (2006) Do Iodinated Contrast Media Interfere with Renal Tubular Creatinine Secretion? Radiology, 240: 615). Moreover, the serum creatinine concentration is not necessarily an accurate reflection of true renal function because the concentration also depends on other factors like age and gender. Furthermore, following an impairment of renal function, the creatinine concentration rises slowly, typically over two or more days. Therefore, an impairment of renal function and, thus, CIN can be only diagnosed delayed when using serum creatinine as a marker. However, the patients who received a contrast medium are frequently not hospitalized anymore at the time when CIN can be diagnosed by measuring creatinine. Therefore, an acute adverse event may occur off hospital bringing putting a patient even at higher risk.

Recently, urotensin II and adiponectin were suggested to play a role in renal physiology and disease. It was shown that urotensin II plasma and urine levels in diabetes patients significantly increased as renal function decreased (Totsune K. et al. (2004) Elevated plasma levels of immunoreactive urotensin II and its increased urinary excretion in patients with Type 2 diabetes mellitus: association with progress of diabetic nephropathy. Peptides. 25(10):1809-14). Moreover, there is evidence that adiponectin may serve as a gender-specific independent predictor of chronic kidney disease progression (Kollerits et al. (2007) Gender-specific association of adiponectin as a predictor of progression of chronic kidney disease: the Mild to Moderate Kidney Disease Study. Kidney Int. 71(12):1279-86). Recently, L-FABP (liver-type fatty acid binding protein) has been reported as a marker for the progression of chronic renal disease and nephrotoxicity (e.g., Kamijo et al.(2006) Urinary liver-type fatty acid binding protein as a useful biomarker in chronic kidney disease. Mol. Cell Biochem. 2006; 284).

Biomarkers which would allow for a reliable diagnosis of CIN are, however, not yet reported but are nevertheless highly desirable.

The technical problem underlying the present invention can be seen as the provision of means and methods for complying with the aforementioned needs. The technical problem is solved by the embodiments characterized in the claims and herein below.

Accordingly, the present invention relates to a method for diagnosing contrast medium-induced nephrotoxicity in a subject, comprising the steps of
a) determining, in a first sample of said subject obtained prior to administering the contrast medium, the amount of urotensin II and/or adiponectin;
b) determining, in a second sample of said subject obtained after administering the contrast medium, the amount of urotensin II and/or adiponectin; and
c) comparing the amount of urotensin II and/or adiponectin as determined in step a) to the respective amount of urotensin II and/or adiponectin as determined in step b), wherein an increase or change of the amount of urotensin II and/or adiponectin as determined in step b) compared with the amount as determined in step a) indicates contrast medium-induced nephrotoxicity.

The method of the present invention, preferably, is an in vitro method. Moreover, it may comprise steps in addition to those explicitly mentioned above. For example, further steps may relate to sample pre-treatments or evaluation of the results obtained by the method. The method of the present invention may be also used for monitoring, confirmation, and subclassification of a subject. The method may be carried out manually or assisted by automation. Preferably, step (a) and/or (b) and/or (c) may in total or in part be assisted by automation, e.g., by a suitable robotic and sensory equipment for the determination in steps (a) and/or (b) or a computer-implemented comparison in step (c).

The term "diagnosing" as used herein means assessing the occurrence or the risk of the occurrence of contrast medium-induced nephrotoxicity in a subject after administration of a contrast medium. As will be understood by those skilled in the art, such an assessment is usually not intended to be correct for all (i.e. 100%) of the subjects to be identified. The term, however, requires that a statistically significant portion of subjects can be identified (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. More preferably, at least 60%, at least 70%, at least 80% or at least 90% of the subjects of a population can be properly identified by the method of the present invention.

Diagnosing according to the present invention includes monitoring, confirmation, subclassification and prediction of the relevant condition or disease, symptoms or risks therefor. Monitoring relates to keeping track of an already diagnosed condition or disease. Confirmation relates to the strengthening or substantiating a diagnosis already performed using other indicators or markers. Subclassification relates to further defining a diagnosis according to different subclasses of the diagnosed condition or disease, e.g. defining according to mild and severe forms of the condition or disease.

The term "contrast medium-induced nephrotoxicity" is known in the art. Preferably, the term relates to an acute impairment of renal function caused by the administration of a contrast medium. Renal function can be, preferably, assessed by means and methods as specified herein below, e.g., by determining the glomerular filtration rate (GFR).

The term "subject" as used herein relates to animals, preferably mammals, and, more preferably, humans. Preferably, the subject referred to in accordance with the method of the present invention is in need of an examination that requires the administration of a contrast medium as laid out elsewhere in this application. Preferably, the subject is an individual without any apparent renal disorder before administering a contrast medium. Renal disorders are known to the person skilled in the art. In this context, the term "renal disorder" is considered to relate to any disease, injury, or dysfunction of the kidney or affecting the kidney, more particularly affecting the capacity of the kidney for waste removal and/or ultrafiltration. Examples for renal disorders include congenital disorders and acquired disorders. Examples for congenital renal disorders include congenital hydronephrosis, congenital obstruction of urinary tract, duplicated ureter, horseshoe kidney, polycystic kidney disease, renal dysplasia, unilateral small kidney. Examples for acquired renal disorders include diabetic or analgesic nephropathy, glomerulonephritis, hydronephrosis (the enlargement of one or both of the kidneys caused by obstruction of the flow of urine), interstitial nephritis, kidney stones, kidney tumors (e.g. Wilms tumor and renal cell carcinoma), lupus nephritis, minimal change disease, nephrotic syndrome (the glomerulus has been damaged so that a large amount of protein in the blood enters the urine. Other frequent features of the nephrotic syndrome include swelling, low serum albumin, and high cholesterol), pyelonephritis, renal failure (e.g. acute renal failure and chronic renal failure). Renal disorders can be diagnosed by any means known and deemed appropriate. Particularly, renal function can be assessed by means of the glomerular filtration rate (GFR). For example, the GFR may be calculated by the Cockgroft-Gault or the MDRD formula (Levey 1999, Annals of Internal Medicine, 461-470). GFR is the volume of fluid filtered from the renal glomerular capillaries into the Bowman's capsule per unit time. Clinically, this is often used to determine renal function. The GFR was originally estimated (the GFR can never be determined, all calculations derived from formulas such as the Cockgroft Gault formula of the MDRD formula deliver only estimates and not the "real" GFR) by injecting inulin into the plasma. Since inulin is not reabsorbed by the kidney after glomerular filtration, its rate of excretion is directly proportional to the rate of filtration of water and solutes across the glomerular filter. In clinical practice however, creatinine clearance is used to measure GFR. Creatinine is an endogenous molecule, synthesized in the body, which is freely filtered by the glomerulus (but also secreted by the renal tubules in very small amounts). Creatinine clearance (CrCl) is therefore a close approximation of the GFR. The GFR is typically recorded in milliliters per minute (mL/min). The normal range of GFR for males is 97 to 137 mL/min, the normal range of GFR for females is 88 to 128 mL/min.

If the GFR has decreased below a critical threshold which allows removal of toxic concentration of uraemia for the blood (usually at a GFR, CrCl < 10-15 mL/min, end-stage renal failure), then - depending also on other clinical circumstances such as the patients clinical condition - renal replacement therapy is indicated, e.g. renal disorders can be diagnosed by any means known and deemed appropriate. Particularly, renal function can be assessed by means of the GFR. One of the first hints for renal disorder is the presence of protein in urine (micro- or macroalbuminuria) which can be assessed by simple dip stick. The most common blood test used to date is still creatinine while acknowledging its missing accuracy.

Preferably, a blood creatinine level, more preferably a serum creatinine level, of larger than 1.2 mg/dl, more preferably of larger than 1.5 mg/dl, and most preferably of larger than 2.0 mg/dl indicates an apparent renal disorder. Accordingly, the subject as referred to in the context of the present invention, preferably, has a blood creatinine level of lower than 1.2 mg/dl, more preferably of lower than 1.5 mg/dl and most preferably of lower than 2.0 mg/dl before a contrast medium is administered.

A GFR of, preferably, lower than 60mL/min, more preferably of lower than 45mL/min , and most preferably of lower than 30mL/min is indicative for an apparent renal disorder. Accordingly, the subject as referred to in the context of the present invention, preferably, showns a GFR (before the administration of a contrast medium) of larger than 60 mL/min, more preferably of larger than 45mL/min , and most preferably of larger than 30 mL/min.

The term "contrast medium" is known by the skilled person. The term, preferably, relates to any substance that is deemed appropriate, when administered to a subject, to increase the contrast, preferably, in X-ray imaging by affecting the attenuation of X-rays, and, therefore, to enhance the visibility of structures and fluids. Thus, a contrast medium, preferably, enables differentiation of certain parts of the body from surrounding areas which are of similar density. Preferably, a contrast medium in the context with the present invention is an opaque or a positive contrast medium, i. e. a contrast medium that has a higher attenuation density than the surrounding tissue and, therefore, an increased absorption of x-rays. Positive contrast media are well known in the art and include, preferably, non iodine based contrast media and, more preferably, iodine based contrast media, i.e. iodinated contrast media. Examples of non iodine based and iodine based contrast media are known by the skilled person. Iodine based contrast media in the context of the present invention, preferably, encompass water insoluble, oily, and more preferably water-soluble iodine based contrast media. Oily iodine based contrast media, preferably, are lipiodol, ethiodol and ethyl esters of iodinated fatty acids of poppy seeds which can be used for lymphography as well as for hysteroalpingography, or iodophylundecyclic acid (iophendylat, Pantopaque, Myodil) which can be used for mylography. Water-soluble iodine based contrast media in the context of the present invention are, preferably, derivatives of tri-iodinated benzoic acid. Derivatives of tri-iodinated benzoic acid are referred to as monomeric when they contain only one benzene ring, and dimeric when they contain two benzene rings. Preferably, the monomeric and the dimeric contrast media as contemplated by the present invention are ionic or, more preferably, non ionic. Most preferably, the contrast medium is a monomeric, non ionic contrast medium such as Iobitridol (Xenetix).
Typically, non ionic contrast media do not dissociate and their water-solubility is due to hydrophilic hydroxyl groups. Monomeric ionic media are salts that dissociate into two molecules, one anion containing the radiopaque property due to three iodine atoms, and one cation without radiopaque properties. They encompass, preferably, oral cholegraphic contrast media and contrast media for urography/angiography. Preferably, monomeric ionic contrast media are ioglicate (Rayvist), iodamide (Uromiro), acetrizoate (Diaginol, Urokon) diatrizoate (Angiografin, Hypaque, Renografin, Urografin, Urovison), metrizoate (Isopaque, Triosil). A variety of different non ionic monomeric contrast media is known in the art. They, preferably, encompass (Amipaque), iohexol (Omnipaque), iopamidol (Iopamiro), iopentol (Imagopaque), iopromide (Ultravist), ioversol (Optiray).

Dimeric iodine based contrast media, preferably, comprise two tri-iodinated benzene rings. They can be grouped in ionic intravenous cholegraphic contrast media, monoacidic ionic contrast media and non ionic contrast media. Dimeric iodinated contrast media are, preferably, sodium meglumine ioxaglate (Hexabrix), iotrolan (Isovist), iodixanol (Visipaque).

Non iodine contrast media frequently contain barium, mainly in form of insoluble barium sulfate. They are, preferably, administered for examining the gastrointestinal tract (e.g. in form of the so called "barium meal" "barium swallow" or in enema form).

Also contemplated by the present invention and also preferred are contrast media based on gadolinium which are also assumed to induce nephrotoxicity (H Akgun et al. Are gadolinium-based contrast media nephrotoxic? A renal biopsy study. Arch Pathol Lab Med 2006; 130:1354). Preferably, contrast media based on gadolinium are used for angiography or for magnetic resonance imaging.

The contrast media may be administered by any method deemed appropriate. The person skilled in the art knows that the method to be selected for administration may depend on the contrast medium and/or the purpose of the examination for which the contrast medium is administered. Preferably, contrast media based on barium are administered by swallowing or in enema form. Contrast media based on iodine are, preferably, administered by injection into the veins, the spinal canal, or the arteries. Also contemplated is the use of a catheter in order to administrate an iodine based contrast medium.

Preferably, the administration of a contrast medium, particularly, of an iodine based contrast medium, is for computed tomography, and, most preferably, for angiography. Further contemplated by the present invention is the administration of a suitable contrast medium for ultrasonic examination or magnetic resonance imaging (MRI). Computed tomography (CT), also referred to as computed axial tomography, is a medical imaging method employing tomography where digital geometry processing is used to generate a three-dimensional image of the internals of a subject from a large series of two-dimensional X-ray images taken around a single axis of rotation. For CT, the contrast medium is, preferably, administered intravenously. Angiography is a medical imaging technique in which a picture (e.g. an X-ray picture or magnetic resonance picture) is taken to visualize the inner opening of blood filled structures, e.g. arteries, veins and the heart chambers. The image of the blood vessels is called an angiograph, or more commonly, an angiogram. For angiography, the contrast medium, preferably, is administered by use of a catheter which is inserted in a peripheral artery, e.g. into a femoral artery. The term "angiography" in the context of the present invention, preferably, relates to cerebral angiography, peripheral angiography, visceral angiography, pulmonary angiography, lymphangiography, right heart ventriculography, left heart ventriculography, aortography, retinal angiography, and, more preferably, to coronary angiography. The term "coronary angiography" is known by the person skilled in the art. A long, thin, flexible tube called a catheter is used to administer a contrast medium at the desired area to be visualized. The catheter is, preferably, threaded into an artery e.g. in the groin or forearm, and the tip is advanced through the arterial system into one of the two major coronary arteries. The angiographic image is typically a shadow picture of the openings within the cardiovascular structures carrying blood (e.g. by means of the contrast medium within the blood). The images may be taken as still images or motion images. Motion images may also show the speed of blood (actually the speed of radiocontrast within the blood) traveling within the blood vessel.

The invention takes advantage of certain biochemical markers. The term "biochemical marker" is known to the person skilled in the art. In particular, biochemical or molecular markers are gene expression products which are differentially expressed (i.e. upregulated or downregulated) in presence or absence of a certain condition, disease, or complication. The level of a suitable biochemical marker can indicate the presence or absence of the condition, disease, or complication, and thus allow diagnosis.

The present invention particularly takes advantage of urotensin II and adiponectin as biochemical markers, either in combination or alone. Additionally, the use of Liver-type fatty acid binding protein as biochemical marker in combination with urotensin II and/or adiponectin is considered in the context of the present invention.

Urotensin II can be found in the spinal cord tissue from many different species (fish, vertebrates, primates) and probably plays a role there as a hormone in the neuro-secretory-system. Urotensin II is a small, cyclic peptide containing a disulfide bridge between two cyteine residues. It was shown that Urotensin II is a ligand for the GPR14 receptor. In humans, urotensin II derives from a larger precursor protein which is referred to as Preprourotensin II. Two isoforms of the urotensin II precursor, 124 and 139 amino acid residues in length, have been identified. (Watanabe T et al. (2006), Human urotensin II as a link between hypertension and coronary artery disease. Hypertens Res.(6):375-87).

Adiponectin is a protein (one of several known adipocytokines) secreted by the adipocyte. Adiponectin exists in a wide range of multimer complexes in plasma and combines via its collagen domain to create 3 major oligomeric forms: a low-molecular weight (LMW) trimer, a middle-molecular weight (MMW) hexamer, and high-molecular weight (HMW) 12- to 18-mer adiponectin (Kadowaki et al.(2006) Adiponectin and adiponectin receptors in insulin resistance, diabetes, and the metabolic syndrome. J Clin Invest. 116(7): 1784-1792). Adiponectin has been reported to have several physiological actions, such as protective activities against atherosclerosis, improvement of insulin sensitivity, and prevention of hepatic fibrosis.

Urotensin II and adiponectin as used herein also encompass variants of the aforementioned specific urotensin II peptides and adiponectin polypeptides, respectively. Such variants have at least the same essential biological and immunological properties as the specific urotensin II peptides and adiponectin polypeptides, respectively. In particular, they share the same essential biological and immunological properties if they are detectable by the same specific assays referred to in this specification, e.g., by ELISA assays using polyclonal or monoclonal antibodies specifically recognizing the urotensin II peptides and adiponectin polypeptides, respectively. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific urotensin II peptides and adiponectin polypeptides, respectively. The degree of identity between two amino acid sequences, in principle, can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific urotensin II peptides and adiponectin polypeptides, respectively, or the aforementioned types of variants as long as these fragments have the essential immunological and biological properties as referred to above. Such fragments may be, e.g., degradation products of the urotensin II peptides and adiponectin polypeptides, respectively. Further included are variants which differ due to posttranslational modifications such as phosphorylation or myristylation. Preferably, urotensin II as used herein also encompasses the precursor proteins of urotensin II such as prepro- Urotensin II.

Adiponectin as used herein, preferably, relates to high molecular weight adiponectin, mid molecular weight adiponection, and, more preferably, to total adiponectin. The terms high molecular weight adiponectin, mid molecular weight adiponectin and total adiponectin are understood by the skilled person.

The term "sample" refers to a sample of a body fluid, to a sample of separated cells or to a sample from a tissue or an organ. Samples of body fluids can be obtained by well known techniques and include, preferably, samples of urine. A urine sample in the context of the present invention may be any urine sample deemed appropriate.

The "first sample" is understood as a sample which is obtained in order to reflect the amount of the respective marker prior to the administration of a contrast medium. Therefore, it is clear to the person skilled in the art that the first sample is, preferably, obtained prior to the administration of a contrast medium or without undue delay after the administration of a contrast medium. Preferably, the "first sample" is obtained shortly before, during or immediately after administration of a contrast medium. Shortly before in this context, preferably, means not more than 3 days, preferably not more than 24 hours, more preferably not more than 8 hours, more preferably not more than 4 hours, more preferably not more than 2 hours, and most preferably not more 1 hour before the administration of a contrast medium. Immediately after in this context, preferably, means not more than 6 hours, more preferably not more than 4 hours, more preferably not more than 2 hour, more preferably not more than 0.5 hours after the administration of a contrast medium, these time-periods being computed from the end of the administration procedure, in case of angiography, preferably, from removing the catheter from the body.

The "second sample" is, preferably, understood as a sample which is obtained in order to reflect a change of the amount of the respective marker as compared to the amount of the respective marker in the first sample. Preferably, the second sample is obtained not too late after administration of a contrast medium (in order to allow timely diagnosis) but also not too early after the first sample (in order to observe a sufficiently significant change to allow for diagnosis). The "second sample" may be obtained within 12 to 48 hours, more preferably within 12 to 36 hours, 12 to 30 hours, 12 to 26 hours, 18 to 26 hours, to 22 to 26 hours, or most preferably 23 to 25 hours after administration of a contrast medium. Preferably, at least one further sample is obtained in order to confirm the change of the amount of a marker. Such further sample may be obtained, preferably, within 24 to 72 hours, and more preferably 18 to 48 hours the administration of a contrast medium.

Determining the amount of the peptides or polypeptides referred to in this specification relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labeled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Nonspecific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labelling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of a marker in a first sample with an amount of said marker in a second samples. The terms "first sample" and "second sample" are specified elsewhere in this description. It is to be understood that comparing as used herein refers to a comparison of corresponding parameters or values. It is to be understood that an amount of a marker is a first sample is compared to an amount of the respective marker, thus the same marker, in a second sample. E.g., the amount of urotensin II in a first sample is compared to the amount of urotensin II in a second sample, or the amount of adiponectin in a first sample is compared to the amount of adiponectin in a second sample. Also contemplated by the present invention is that the determined amounts are normalized for carrying out the comparison step. Preferably, the normalization is done by relating the amount of the respective biomarker, i.e. of adiponectin, urotensin II or L-FABP in a sample of a subject to the amount of creatinine in said sample, more preferably, by determining the ratio of the amount of the respective marker to the amount of creatinine. Accordingly, the comparison step is carried out by comparing the thus determined ratios for the respective marker.

Preferably, the diagnosis is based on the comparison of the amount of a marker of the present invention in a first sample obtained prior to the administration of a contrast medium to the amount of the respective marker in a second sample obtained after administration of said contrast medium. Also mentioned above, also the comparison of normalized amounts is contemplated by the present invention. Preferably, an increase or change, and more preferably a statistically significant increase or change of urotensin II or adiponectin or both is indicative for contrast medium-induced nephrotoxicity.

Particularly, a significant increase is an increase of a size which is considered to be significant for diagnosis, particularly statistically significant. The terms "significant" and "statistically significant" are known to the person skilled in the art. Whether a change or an increase is statistically significant can be determined without further ado by the person skilled in the art using various well known statistic evaluation tools including those referred to herein.

Preferred significant increases of the amounts of urotensin II and adiponectin in urine samples which have been found in the course of the invention to be associated with the diagnosis or risk of cardiac medium-induced nephrotoxicity are given below.

According to the invention, an increase of the amount of urotensin II in the second sample compared to the amount in the first sample, preferably, of at least 100 %, more preferably of at least 300 %, and even, more preferably, of at least 500 %, and most preferably of at least 800 % is considered to be significant and, thus, to be associated with the occurrence or the risk of contrast medium-induced nephrotoxicity in a subject after administration of a contrast medium.

According to the invention, an increase of the amount of adiponectin in the second sample compared to the amount in the first sample, preferably, of at least 100 %, more preferably of at least 300 % , and even more preferably of at least 500 %, and most preferably of at least 800 % is considered to be significant and, thus, to be associated with the occurrence or the risk of contrast medium-induced nephrotoxicity in a subject after administration of a contrast medium.

Once the contrast medium-induced nephrotoxicity or a risk thereof has been diagnosed, a suitable treatment can be initiated (e.g. hydration, administration of N-acetylcysteine, volume supplementation by sodium bicarbonate, administration of ascorbic acid).

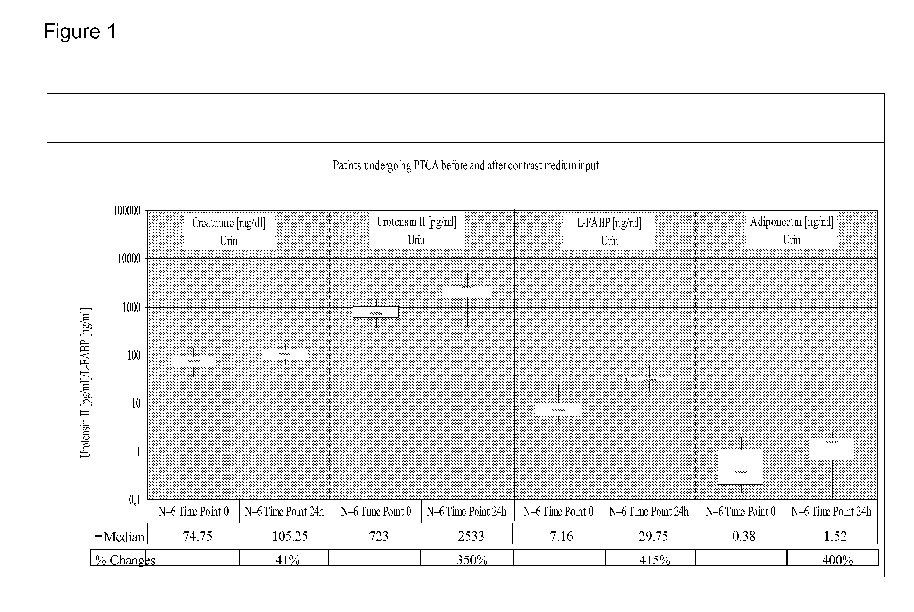

Advantageously, it has been found in the studies underlying the present invention that determining, in a first sample of subject obtained prior to administering a contrast medium, the amount of urotensin II and/or adiponectin, and determining, in a second sample of said subject obtained after administering the contrast medium the amount of urotensin II and/or adiponectin, and comparing the amount of urotensin II and/or adiponectin in said first sample to the amount of urotensin II and/or adiponectin in said second sample, is required for diagnosing contrast medium-induced nephrotoxicity in a subject after administration of a contrast medium. In the studies underlying the present invention a dose response analysis was carried out in order to investigate whether there is an effect of the administered volume of a contrast medium (the dose) on the amount of urotensin II, adiponectin and U-L-FABP. The dose response analysis was based on the principle that there is a relationship between a toxic reaction (here a nephrotoxic reaction) and the dose of a toxic substance (here the contrast medium) received. Surprisingly, it was shown that amounts of adiponectin and L-FABP were significantly increased after the administration of a high dose of a contrast medium, whereas the effect of lower doses of a contrast medium was lower (lower increase or even decrease, see Examples). Moreover, the median values in the examined patient population (including patients which received high and low doses) for urotensin II, adiponectin and L-FABP in urine were significantly increased in the examined patient population 24 h after administration of a contrast medium strongly indicating that the administration of a contrast medium influences the concentration of these markers (see Fig. 1). The observed effects were even stronger than for creatinine (see also Fig. 1).

Particularly, the method of the present invention will, if applied, allow an early and reliable diagnosis of contrast medium-induced nephrotoxicity. The methods described in the prior art for the diagnosis do not disclose a method for diagnosing contrast medium-induced nephrotoxicity based on urotensin II and adiponectin. The method of the present invention is particularly advantageous since it allows an early diagnosis of cardiac induced nephrotoxicity, e.g. already after a single day of the administration of a contrast medium. Thus, it is possible to start an appropriate treatment earlier than previously possible. Since it is well known in the art that patients experiencing contrast medium-induced nephrotoxicity generally have more complications, more serious long term outcomes and a prolonged hospital stay than patients without nephrotoxicity, the method of the present invention may allow to improve the general condition of patients with contrast medium-induced nephrotoxicity, and, thus, may be particularly beneficial to the health system.

Moreover, the method of the present invention is advantageous since a patient frequently is still hospitalized one day after the administration of a contrast medium, whereas after two days he might already have left the hospital. Therefore, the risk of an acute adverse event that occurs off hospital may be significantly reduced when applying the method of the present invention.

In a preferred embodiment of the method of the present invention, additionally the amount of L-FABP (liver-type fatty acid binding protein), or a variant thereof, is determined in the first sample and second sample of said subject. Preferably, the L-FABP is urinary L-FABP (U-L-FABP). Preferably, the amount of L-FABP in the first sample is compared with the amount of L-FABP in the second sample. More preferably, an increase, particularly a significant increase, of the amount of L-FABP in the second sample compared to the amount of L-FABP in the first sample indicates contrast medium-induced nephrotoxicity.

Liver-type fatty acid binding protein is an intracellular carrier protein of free fatty acids that is expressed in the proximal tubules of the human kidney. Kamijo et al.(Urinary liver-type fatty acid binding protein as a useful biomarker in chronic kidney disease. Mol. Cell Biochem. 2006; 284) reported that urinary excretion of L-FABP may reflect various kind of stresses that cause tubulointerstitial damage and may be a useful clinical marker of the progression of chronic renal disease. Moreover, there is evidence that the level of urinary L-FABP is a predictive marker for contrast medium-induced nephrotoxicity. Nakumura et al. showed that the amount of urinary L-FABP increased significantly in patients with contrast medium-induced nephropathy (Nakamura et al. (2006) Urinary excretion of liver-type fatty acid-binding protein in contrast medium-induced nephropathy. Am J Kidney Dis.; 47(3) :439-44.)

According to the invention, an increase of the amount of L-FABP in the second sample compared to the amount in the first sample, preferably, of at least 100 %, more preferably of at least 300 %, and, most preferably, of at least 500 % is considered to be significant and, thus, to be associated with the occurrence or the risk of contrast medium-induced nephrotoxicity in a subject after administration of a contrast medium.

Moreover, the present invention also relates to the use ofurotensin II and/or adiponectin in a sample of a subject for assessing biocompatibility of a contrast medium.

The term "biocompatibility" refers to the ability of a contrast medium to be administered to a patient and not cause toxic effects, particularly nephrotoxic effects. The lower the toxic effect caused by a contrast medium, the more biocompatible is a contrast medium, and vice versa. Therefore, it can be assessed whether a contrast medium shall be used or not for imaging. The contrast media to be assessed may be any contrast medium deemed appropriate. Moreover, examples for contrast media are given herein above.

Moreover the present invention also relates to the use of urotensin II and/or adiponectin in a sample of a subject for diagnosing contrast medium-induced nephrotoxicity after angiography.

The present invention, furthermore, relates to a device for diagnosing contrast medium-induced nephrotoxicity in a subject comprising
a) means for determining the amount of urotensin II and/or adiponectin in a sample of a subject; and
b) means for comparing the amount in a first sample determined by the means of a) with the respective amount in a second sample determined by the means of a), whereby diagnosis of contrast medium-induced nephrotoxicity in a subject after administration of a contrast medium is allowed.

The term "device" as used herein relates to a system of means comprising at least the aforementioned means operatively linked to each other as to allow the diagnosis. Preferred means for determining the amount of urotensin II and means for determining the amount of a adiponectin, and means for comparing an amount in a first sample to an amount in a second sample and means for diagnosing contrast medium-induced nephrotoxicity after administration of a contrast medium are disclosed herein in connection with the method of the invention. Further comprised by the device of the present invention are, preferably, means for determining the amount of L-FABP in a sample of a subject and means for comparing the amount of L-FABP in a first sample to the amount of L-FABP in a second sample. How to link the means in an operating manner will depend on the type of means included into the device. For example, where means for automatically determining the amount of the peptides are applied, the data obtained by said automatically operating means can be processed by, e.g., a computer program in order to obtain the desired results. Preferably, the means are comprised by a single device in such a case. Said device may accordingly include an analyzing unit for the measurement of the amount of the peptides or polypeptides in an applied sample and a computer unit for processing the resulting data for the evaluation. Alternatively, where means such as test stripes are used for determining the amount of the peptides or polypeptides, the means for comparison may comprise control stripes or tables allocating the determined amount to a reference amount. The test stripes are, preferably, coupled to a ligand which specifically binds to the peptides or polypeptides referred to herein. The strip or device, preferably, comprises means for detection of the binding of said peptides or polypeptides to the said ligand. Preferred means for detection are disclosed in connection with embodiments relating to the method of the invention above. In such a case, the means are operatively linked in that the user of the system brings together the result of the determination of the amount and the diagnostic or prognostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing urotensin II or adiponectin, Plasmon surface resonance devices, NMR spectrometers, mass- spectrometers etc.) or evaluation units/devices referred to above in accordance with the method of the invention.

Finally, the present invention relates to a kit adapted to carry out the method of the present invention, said kit comprising instructions for carrying out the said method, and
a) means for determining the amount of urotensin II and/or adiponectin in a sample of a subject; and
b) means for comparing the amount in a first sample determined by the means of a) with the respective amount in a second sample determined by the means of a), whereby diagnosis of contrast medium-induced nephrotoxicity is allowed.

The term "kit" as used herein refers to a collection of the aforementioned means, preferably, provided in separately or within a single container. Moreover, and also preferred, the kit may comprise means to determine the amount of L-FABP in a sample of a subject and means for comparing the amount of L-FABP in a first sample to the amount of L-FABP in a second sample. The container, also preferably, comprises instructions for carrying out the method of the present invention. Accordingly, a kit adopted for carrying out the method of the present invention comprises all components required for practicing said method in an ready-to-use manner, e.g., in a premixed form with adjusted concentrations of the components used for determination and/or comparison.

All references cited in this specification are herewith incorporated by reference with respect to their entire disclosure content and the disclosure content specifically mentioned in this specification.

### The figure shows:

Fig. 1 Box-plot: The amount of creatinine, urotensin II, L-FABP and adiponectin were determined in urine samples of subjects undergoing percutaneous transluminal coronary angioplasty (PTCA) before (time point 0) and 24 h after (time point 24 h) administration of a contrast medium. A total of six individuals was included in the study, each receiving a different amount of a contrast medium (50 ml, 80 ml, 100 ml, 150 ml, 220 ml and 350 ml, respectively). The median values of urotensin II, L-FABP and adiponectin were significantly increased 24 h after contrast medium administration (350%, 415% and 400%), whereas creatinine was only increased slightly (41 %).

The following Examples shall merely illustrate the invention. They shall not be construed, whatsoever, to limit the scope of the invention.

### Example 1:

Dose response analysis of the effect of a contrast medium on the level of creatinine, urotensin II, total adiponectin and U-L-FABP in patients undergoing PCTA.

The effect of the administration of various amounts of the contrast medium Iobitridol (Xenetix, Xen 350) on the on the level of creatinine, urotensin II, total adiponectin and L-FABP in urine samples obtained from subject which underwent percutaneous transluminal coronary angioplasty (PCTA) was analysed. All six patients examined in this study had no apparent renal disorder indicated by a serum creatinine level of less than 1.2 mg/dl (before PCTA). Each patient received a different amount of the contrast medium (50 ml, 80 ml, 100 ml, 150 ml, 220 ml and 350 ml, respectively). The urinary levels of creatinine, urotensin II, total adiponectin and L-FABP were determined in samples that were obtained prior to the administration of different volumes of the contrast medium (time point 0) and 24 h after the administration of the contrast medium. The concentration of total Adiponectin was determined by using the Adiponectin EIA Kit (Alpco Diagnostics (Salem, NH, USA), Urotensin II was determined by using an urotensin II kit from Immundiagnostik (Germany), and L-FABP by using the Human L-FABP Elisa Kit (CMIC, Japan). The median values for the measured markers are shown in Fig 1. The median values of urotensin II, L-FABP and adiponectin were significantly increased 24 h after contrast medium administration (350%, 415% and 400%) compared to the increase of creatinine (41 %). Table 1 shows the amounts of L-FABP and adiponectin for the individual examined patients. The results are normalized to the urinary creatinine concentration. From the data it clearly can be seen that a high dose of an administered contrast medium leads to a significant increase of adiponectin and L-FABP.

**Table 1: Adiponectin and L-FABP amounts prior (t= 0 h) and 24 h after contrast medium administration. The amounts were normalized/related to urinary creatinine (Crea).**

| Patient No. | Contrast Medium | 0h Adiponectin | 24h Adiponectin | Changes | 0h L-FABP | 24h L-FABP | Changes |
|---|---|---|---|---|---|---|---|
| | ml Xen350 | ug/g Crea | ug/g Crea | % | ug/g Crea | ug/g Crea | % |
| 1 | 50 | 0.92 | 0.11 | -88% | 20.19 | 11.17 | -45% |
| 2 | 80 | 2.99 | 2.34 | -22% | 42.71 | 63.48 | 49% |
| 3 | 100 | 2.68 | 1.49 | -44% | 13.77 | 19.33 | 40% |
| 4 | 150 | 0.32 | 0.03 | -91 % | 6.8 | 32.74 | 481% |
| 5 | 220 | 0.41 | 3.68 | 897% | 13.1 | 71.23 | 544% |
| 6 | 350 | 0.1 | 0.69 | 690% | 4.36 | 17.71 | 406% |

## Claims

1. A method for diagnosing contrast medium-induced nephrotoxicity in a subject, comprising the steps of
a) determining, in a first sample of said subject obtained prior to administering the contrast medium, the amount ofurotensin II and/or adiponectin;
b) determining, in a second sample of said subject obtained after administering the contrast medium, the amount of urotensin II and/or adiponectin; and
c) comparing the amount of urotensin II and/or adiponectin as determined in step a) to the respective amount of urotensin II and/or adiponectin as determined in step b), wherein a increase of the amount of urotensin II and/or adiponectin as determined in step b) compared with the amount as determined in step a) indicates contrast medium-induced nephrotoxicity.

2. The method of claim 1, wherein the subject prior to administering the contrast medium does not have an apparent renal disorder.

3. The method of claims 1 and 2, wherein the said second sample has been obtained 12 to 48 hours after administering the contrast medium.

4. The method of any one of claims 1 and 3, wherein the contrast medium is non-ionic or ionic.

5. The method of any one of claims 1 to 4, wherein the contrast medium is a iodine-based contrast medium.

6. The method of any one of claims 1 to 5, wherein the sample is urine.

7. The method of any one of claims 1 to 6, wherein the contrast medium has been administered for angiography.

8. The method of claim 7, wherein the angiography is coronary angiography.

9. The method of any one of claims 1 to 8, wherein the increase for urotensin II is at least 300 %.

10. The method of any one of claims 1 to 8, wherein the increase for adiponectin is at least 300 %.

11. The method of any one of claims 1 to 10, wherein in addition in the said first sample and the said second sample the amount of L-FABP (liver-type fatty acid binding protein) is determined.

12. The method of claim 11, wherein the amount of L-FABP in the said first sample is compared with the amount of L-FABP in the said second sample and wherein an increase of the amount of L-FABP in the said second sample compared with the said first sample indicates contrast medium-induced nephrotoxicity.

13. The method of any one of claims 1 to 12, wherein the subject is human.

14. Use of urotensin II and/or adiponectin in a sample of a subject for assessing biocompatibility of a contrast medium.

15. Use of urotensin II and/or adiponectin in a sample of a subject for diagnosing contrast medium-induced nephrotoxicity after angiography.

16. A device for diagnosing contrast medium-induced nephrotoxicity in a subject comprising
a) means for determining the amount of urotensin II and/or adiponectin in a sample of a subj ect; and
b) means for comparing the amount in a first sample determined by the means of a) with the respective amount in a second sample determined by the means of a), whereby diagnosis of contrast medium-induced nephrotoxicity is allowed.

17. A kit adapted to carry out the method of any one of claims 1 to 12, said kit comprising instructions for carrying out the said method, and
a) means for determining the amount of urotensin II and/or adiponectin in a sample of a subj ect; and
b) means for comparing the amount in a first sample determined by the means of a) with the respective amount in a second sample determined by the means of a), whereby diagnosis of contrast medium-induced nephrotoxicity is allowed.
